# EUROPÄISCHE PATENTSCHRIFT

(11) **EP 0 955 037 B1**
(45) Veröffentlichungstag und Bekanntmachung des Hinweises auf die Patenterteilung: **12.05.2004**
(21) Anmeldenummer: 99105727.4
(22) Anmeldetag: 20.03.1999
(51) Int. Cl.: A61K 7/48, A61K 7/50

(54) **Hautpflegemittel**
Skin care compositions
Compositions pour le soin de la peau

(30) Priorität: 30.03.1998 DE 19814065
(43) Veröffentlichungstag der Anmeldung: 10.11.1999
(73) Patentinhaber: Cognis Deutschland GmbH & Co. KG, 40589 Düsseldorf (DE)
(72) Erfinder: LeHen Ferrenbach, Catherine, 77100 Meaux (FR); Robbe-Tomine, Laurence, 77330 Ozoir-La-Ferriere (FR); Westfechtel, Alfred, Dr., 40724 Hilden (DE)

(56) Entgegenhaltungen:
- EP-A- 0 008 105
- EP-A- 0 282 289
- EP-A- 0 651 990
- WO-A-95/05160
- WO-A-99/04756
- US-A- 3 098 795

## Beschreibung

### Gebiet der Erfindung

Die Erfindung betrifft Hautreinigungsmittel mit einem Gehalt an ausgewählten Estern als Ölkörper sowie die Verwendung der Ester zur Herstellung von Hautpflegemitteln, insbesondere von Abschminkmitteln in Emulsionsform.

### Stand der Technik

Im Bereich der dekorativen Kosmetik finden die unterschiedlichsten Einsatzstoffe Verwendung. Eine typische Make-up-Zusammensetzung enthält beispielsweise Glycerinmonostearat, Cetylalkohol, Stearinsäure, Paraffinöl, Cetylstearyloctanoat, Octylpalmitat, Talkum, Titandioxid, Eisenoxide, Propylenglycol, Polysorbate, Xanthan, Magnesium-Aluminiumsilicat, Glycerin, Parfümöle, Konservierungsmittel und Wasser. In Wimperntusche oder Mascara sind Wachse, Farbstoffe, Emulgatoren und Verdickungsmittel enthalten. Lippenstifte enthalten neben Farbstoffen, Wachsen und Ölkörpern vor allem auch Glimmer Titandioxid und neuerdings auch Siliconverbindungen. Lidschatten stellen üblicherweise Mischungen von Farbstoffen mit Glimmer oder Titandioxid dar, die als weitere Bestandteile Talkum, Paraffinöl, Kaolin, Metallseifen, Wachsalkohole und Emulgatoren enthalten können. Kajalstifte enthalten beispielsweise Eisenoxide, Pflanzenöle, Wachse, Fettsäuren, Talkum und Emulgatoren. Entsprechende kosmetische Reinigungsmittel müssen somit eine Vielzahl völlig unterschiedlicher Stoffe möglichst vollständig von der Haut entfernen, also typische Wachse, Öle und Siliconverbindungen lösen und gleichzeitig auch Pigmente wie Glimmer oder Titandioxid solubilisieren. Des weiteren müssen sie so mild wie nur eben möglich sein, um bei den Verbraucherinnen keine Hautrötungen oder gar Augenschleimhautirritationen auszulösen. Üblicherweise werden derartige Zubereitungen, wie sie beispielsweise in der **EP 0705592 B1** (L'Oréal) offenbart sind, entweder in Form von Lotionen, gegebenenfalls auf Watteträgern, oder als Cremes angeboten. Hier besteht seitens der Verbraucherin zusätzlich der Wunsch, daß die Produkte, bei denen es sich stets um Emulsionen handelt, eine ausreichende Lagerstabilität besitzen und sich insbesondere nicht in der Wärme irreversibel entmischen.

Die **EP 651 990 A1** offenbart Abschminkmittel, die Ester mit 12 bis 20 Kohlenstoffatomen enthalten und vorzugsweise eine spezielle Emulgator-Kombination. Die **WO 95/05160 A1** betrifft schäumende Reinigungszusammensetzungen mit einem Gehalt an substituierten Alkanoaten (u. a. Lactate) sowie anionischen oder amphoteren Tensiden. Die **EP 008 105 A2** betrifft die Verwendung spezifischer Citronensäureester als flüssige Ölkomponente in kosmetischen Zubereitungen, u. a. in einer O/W-Reinigungsmilch. Die EP 282 289 A1 offenbart Zusammensetzungen, die Di-Kationensalze von Monoestem der Citronensäure enthalten. Die **US 3,098,795** beschreibt kosmetische Zubereitungen auf Basis von wasserfreiem Lanolin, einem pflanzlichen Öl und einem Ester eines unverzweigten C₈-C₁₈ Fettalkohols und Milchsäure.

Demzufolge hat die komplexe Aufgabe der vorliegenden Erfindung darin bestanden, neue Hautreinigungsmittel in Emulsionsform zur Verfügung zu stellen, die ein hohes Reinigungsvermögen sowohl gegenüber Wachsen, Ölen, Siliconverbindungen als auch gegenüber Pigmenten aufweisen, außerordentlich haut- und schleimhautverträglich sind und gleichzeitig auch eine besonders hohe Lagerstabilität besitzen.

### Beschreibung der Erfindung

Gegenstand der Erfindung sind wäßrige Hautreinigungsmittel, enthaltend
(a) 10 bis 90 Gew.-% Ölkörper vom Typ der Ester mehrwertiger und/oder hydroxyfunktionalisierter Carbonsäuren ausgewählt aus der Gruppe Bernsteinsäure, Maleinsäure, Itaconsäure, Adipinsäure, Sebacinsäure, Dodecandisäure und/oder Äpfelsäure, Weinsäure und Fettalkoholen mit 12 bis 18 Kohlenstoffatomen und
(b) 1 bis 15 Gew.-% Emulgatoren.

Überraschenderweise wurde gefunden, daß die Ester, die die Komponente (a) bilden, gegenüber konventionellen Ölkörpem ein wesentlich verbessertes Reinigungsvermögen besitzen und dabei besonders hautschonend sind. In ihrer Wirksamkeit gegenüber Produkten des Stands der Technik, die als Ölkörper konventionelle Fettsäuremethylester enthalten, erreichen die Ölkörper der Komponente (a) auf einer Skala von 1 bis 100 %, mit der die Entfernung komplexer kosmetischer Anschmutzungen von der Haut beurteilt wird, Werte von 65 bis 80 % (zum Vergleich: Rapsfettsäuremethylester: 40 %). Die erfindungsgemäßen Mittel zeichnen sich des weiteren auch durch eine besondere Stabilität bei Temperaturlagerung aus.

### Ester

Ester, die als Komponente (a) der erfindungsgemäßen Zubereitungen in Betracht kommen, leiten sich ab von Bernsteinsäure, Maleinsäure, ltaconsäure, Adipinsäure, Sebacinsäure, Dodecandisäure, Äpfelsäure, und Weinsäure. Die Alkoholkomponente der Ester weist 12 bis 18 Kohlenstoffatome auf. Typischerweise handelt es sich dabei um Fettalkohole, wie beispielsweise Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind Voll- und/oder Partialester der Maleinsäure und Weinsäure mit technischen Fettalkoholen mit 12 bis 18 Kohlenstoffatomen, wie beispielsweise Kokos-, Palm-, Palmkem- oder Talgfettalkohol. Die Einsatzmenge der Ester kann im Bereich von 10 bis 90, vorzugsweise 15 bis 75 und insbesondere 25 bis 50 Gew.-% - bezogen auf die Zubereitungen liegen.

### Emulgatoren

Als Emulgatoren kommen beispielsweise **nichtionogene Tenside** aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **anionische Emulgatoren** sind Alkylethersulfate, Fettsäuremonoglyceridsulfate, Fettsäureestersulfate, Fettsäureisethionate und Proteinfettsäurekondensate, insbesondere solche auf Basis von Weizen- oder Reisproteinen. Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Coc*amidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkyl-gruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch **kationische Emulgatoren** in Betracht, wobei solche vom Typ der Esterquats, vorzugsweise methylquatemierte Difettsäuretriethanolaminester-Salze, besonders bevorzugt sind.

Der Emulgatoranteil liegt in der Regel in der Größenordnung von 1 bis 15, vorzugsweise 2 bis 10 und insbesondere 5 bis 8 Gew.-% - bezogen auf die Emulsionen, weiche ihrerseits einen wäßrigen Anteil von 10 bis 95, vorzugsweise 25 bis 90 und insbesondere 50 bis 75 Gew.-% aufweisen können.

### Gewerbliche Anwendbarkeit

Im Sinne der Erfindung handelt es sich bei den Hautreinigungsmitteln vorzugsweise um Abschminkmittel, die in Form von W/O-, insbesondere aber als O/W-Emulsionen vorliegen können. Typischerweise enthalten die Mittel
(a) 10 bis 90, vorzugsweise 15 bis 75 Gew.-% Ölkörper vom Typ der Ester mehrwertiger und/oder hydroxyfunktionalisierter Carbonsäuren und
(b) 1 bis 15, vorzugsweise 5 bis 10 Gew.-% ionische und/oder nichtionische Emulgatoren,
mit der Maßgabe, daß sich die Mengenangaben mit Wasser und gegebenenfalls weiteren Hilfs- und Zusatzstoffen zu 100 Gew.-% ergänzen. Die Ester weisen dabei in einer bevorzugten Ausführungsform der Erfindung ein Molekulargewicht im Bereich von 300 bis 500 Dalton auf.

### Hilfs- und Zusatzstoffe

Ferner können die Zubereitungen als weitere Hilfs- und Zusatzstoffe milde Tenside, zusätzliche Ölkörper, Überfettungsmittel, Perlglanzwachse, Stabilisatoren, Konsistenzgeber, Verdickungsmittel, Polymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen. Der Tensidanteil liegt beispielsweise im Bereich von 5 bis 15 Gew.-% - bezogen auf die Zubereitungen.

Als zusätzliche **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe in Betracht. Die Menge an weiteren Ölkörpem kann 10 bis 50 Gew.-% - bezogen auf die Ester der mehrwertigen und/oder hydroxyfunktionalisierten Carbonsäuren - ausmachen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinyl-imidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A1** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere** und **nichtionische Polymere** kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cydische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester,
- Ester der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P.Finkel in SÖFW-Journal 122, 543 (1996) zu entnehmen.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methyl-cedrylketon, zu den Alkoholen Anethol, Citronellol, Eugenol, lsoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, ∝-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

Ein weiterer Gegenstand der Erfindung betrifft die Verwendung von Estern mehrwertiger und/oder hydroxyfunktionalisierter Carbonsäuren mit Fettalkoholen mit 6 bis 22 Kohlenstoffatomen als Ölkörper in Hautreinigungsmitteln, in denen sie in Mengen von 10 bis 90, vorzugsweise 15 bis 75 Gew.-% - bezogen auf die Zubereitungen - enthalten sein können.

### Beispiele

Die nachstehenden Rezepturbeispiele verstehen sich mit Wasser ad 100 Gew.-%.

| **Reinigungscreme** | |
|---|---|
| Cetearyl Glucoside | 5,0 |
| Polyglyceryl-3 Diisostearate | 4,0 |
| Myristyl Adipate | 10,0 |
| Bees Wax | 3,0 |
| Dicaprylyl Ether | 5,0 |
| Glycerol (86 Gew.-%ig) | 5,0 |
| Magnesium Sulfate | 1,0 |

| **Reinigungsemulsion für die Haut** | |
|---|---|
| Cetearyl Glucoside | 5,0 |
| Glyceryl Stearate (and) Ceteareth-12/20 (and) Cetearyl alcohol (and) Cetyl Palmitate | 5,0 |
| Potassium Coco Fatty Acid Hydrolyzed Wheat Protein | 2,0 |
| Almond Oil | 20,0 |
| Bisoctyl Maleate | 10,0 |
| Cetearyl Isononanoate | 3,0 |
| Propylenglycol | 3,0 |

## Patentansprüche

1. Wäßrige Hautreinigungsmittel, enthaltend
(a) 10 bis 90 Gew.-% Ölkörper vom Typ der Ester mehrwertiger und/oder hydroxyfunktionalisierter Carbonsäuren ausgewählt aus der Gruppe Bemsteinsäure, Maleinsäure, Itaconsäure, Adipinsäure, Sebacinsäure, Dodecandisäure und/oder Äpfelsäure, Weinsäure und Fettalkoholen mit 12 bis 18 Kohlenstoffatomen und
(b) 1 bis 15 Gew.-% Emulgatoren.

2. Mittel nach den Anspruch 1, **dadurch gekennzeichnet, daß** sie nichtionische Emulgatoren enthalten, die ausgewählt sind aus der Gruppe, die gebildet wird von Anlagerungsprodukten von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C_{12/18}-Fettsäuremono- und -diestem von Anlagerungsprodukten von 1 bis 30 Mol Ethylenoxid an Glycerin; Glycerinmono- und -diestem und Sorbitanmono- und -diestem von gesättigten und ungesättigten Fettsäuren mit 6 bis 22 Kohlenstoffatomen und deren Ethylenoxidanlagerungsprodukten; Alkylmono- und -oligoglycosiden mit 8 bis 22 Kohlenstoffatomen im Alkylrest und deren ethoxylierte Analoga; Anlagerungsprodukten von 15 bis 60 Mol Ethylenoxid an Ricinusöl und/oder gehärtetes Ricinusöl; Polyolestem; Anlagerungsprodukten von 2 bis 15 Mol Ethylenoxid an Ricinusöl und/oder gehärtetes Ricinusöl; Partialestem auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkoholen, Alkylglucosiden sowie Polyglucosiden; Mono-, Di- und Trialkylphosphaten sowie Mono-, Di- und/oder Tri-PEG-alkylphosphaten und deren Salzen; Wollwachsalkoholen; Polysiloxan-Polyalkyl-Polyether-Copolymeren; Mischestem aus Pentaerythrit, Fettsäuren, Citronensäure und Fettalkohol und/oder Mischestem von Fettsäuren mit 6 bis 22 Kohlenstoffatomen, Methylglucose und Polyolen sowie Polyalkylenglycolen.

3. Mittel nach den Ansprüchen 1 bis 2, **dadurch gekennzeichnet, daß** sie ionische Emulgatoren enthalten, die ausgewählt sind aus der Gruppe, die gebildet wird von Alkylethersulfaten, Fettsäuremonoglyceridsulfaten, Fettsäureestersulfaten, Fettsäureisethionaten, Proteinfettsäurekondensaten, Betainen und Esterquats.

4. Verwendung von Ester mehrwertiger und/oder hydroxyfunktionalisierter Carbonsäuren ausgewählt aus der Gruppe Bernsteinsäure, Maleinsäure, Itaconsäure, Adipinsäure, Sebacinsäure, Dodecandisäure und/oder Äpfelsäure, Weinsäure und Fettalkoholen mit 12 bis 18 Kohlenstoffatomen als Ölkörper in Hautreinigungsmitteln.

## Claims

1. Water-based skin cleansing preparations containing
(a) 10 to 90% by weight of oil components in the form of polybasic and/or hydroxyfunctionalized carboxylic acids selected from the group consisting of succinic acid, maleic acid, itaconic acid, adipic acid, sebacic acid, dodecanedioic acid and/or malic acid, tartaric acid and C₁₂₋₁₈ fatty alcohols and
(b) 1 to 15% by weight of emulsifiers.

2. Preparations as claimed in claim 1, **characterized in that** they contain nonionic emulsifiers selected from the group consisting of products of the addition of 2 to 30 mol ethylene oxide and/or 0 to 5 mol propylene oxide onto linear fatty alcohols containing 8 to 22 carbon atoms, onto fatty acids containing 12 to 22 carbon atoms and onto alkylphenols containing 8 to 15 carbon atoms in the alkyl group; C_{12/18} fatty acid monoesters and diesters of products of the addition of 1 to 30 mol ethylene oxide onto glycerol; glycerol monoesters and diesters and sorbitan monoesters and diesters of saturated and unsaturated fatty acids containing 6 to 22 carbon atoms and ethylene oxide addition products thereof; alkyl mono- and oligoglycosides containing 8 to 22 carbon atoms in the alkyl moiety and ethoxylated analogs thereof; products of the addition of 15 to 60 mol ethylene oxide onto castor oil and/or hydrogenated castor oil; polyol esters; products of the addition of 2 to 15 mol ethylene oxide onto castor oil and/or hydrogenated castor oil; partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols, alkyl glucosides and polyglucosides; mono-, di- and trialkyl phosphates and mono-, di- and/or tri-PEG-alkyl phosphates and salts thereof; wool wax alcohols; polysiloxane/polyalkyl polyether copolymers; mixed esters of pentaerythritol, fatty acid, citric acid and fatty alcohol and/or mixed esters of fatty acids containing 6 to 22 carbon atoms, methyl glucose and polyols and polyalkylene glycols.

3. Preparations as claimed in claims 1 and 2, **characterized in that** they contain ionic emulsifiers selected from the group consisting of alkyl ether sulfates, fatty acid monoglyceride sulfates, fatty acid ester sulfates, fatty acid isethionates, protein fatty acid condensates, betaines and esterquats.

4. The use of esters of polybasic and/or hydroxyfunctionalized carboxylic acids selected from the group consisting of succinic acid, maleic acid, itaconic acid, adipic acid, sebacic acid, dodecanedioic acid and/or malic acid, tartaric acid and fatty alcohols containing 12 to 18 carbon atoms as oil components in skin cleansing preparations.

## Revendications

1. Compositions de nettoyage de la peau aqueuses contenant :
(a) de 10 à 90 % en poids de composés huileux du type des esters d'acide carboxylique plurifonctionnel et/ou fonctionnalisé par un hydroxy choisi dans le groupe de l'acide succinique, de l'acide maléique, de l'acide itaconique, de l'acide adipique, de l'acide sébacique, de l'acide dodécanedioïque et/ou de l'acide malique, de l'acide tartrique et d'alcools gras ayant de 12 à 18 atomes de carbone, et
(b) de 1 à 15 % en poids d'agents émulsionnants.

2. Compositions selon la revendication 1,
**caractérisée en ce qu'**
elles contiennent des agents émulsionnants non ioniques qui sont choisis dans le groupe qui est formé de produits d'addition de 2 à 30 mol d'oxyde d'éthylène et/ou de 0 à 5 mol d'oxyde de propylène sur des alcools gras linéaires ayant de 8 à 22 atomes de carbone, sur des acides gras ayant de 12 à 22 atomes de carbone et sur des alkylphénols ayant de 8 à 15 atomes de carbone dans le groupe alkyle ; des mono- et des diesters d'acide gras en C₁₂/C₁₈ de produits d'addition de 1 à 30 mol d'oxyde d'éthylène sur le glycérol ; des mono- et des diesters de glycérol et des mono- et des diesters de sorbitanne d'acides gras saturés et non saturés ayant de 6 à 22 atomes de carbone et leurs produits d'addition de l'oxyde d'éthylène ; des mono et des oligo-alikylglycosides ayant de 8 à 22 atomes de carbone dans le reste alkyle et leurs analogues éthoxylés ; des produits d'addition de 15 à 60 mol d'oxyde d'éthylène sur l'huile de ricin et/ou l'huile de ricin durcie ; des esters de polyol ; des produits d'addition de 2 à 15 mol d'oxyde d'éthylène sur de l'huile de ricin et/ou l'huile de ricin durcie ; des esters partiels à base d'acides gras en C₆/C₂₂, linéaires, ramifiés non saturés ou saturés, de l'acide ricinoléique ainsi que de l'acide 12-hydroxystéarique et du glycérol, du polyglycérol, du pentaerythritol, du dipentaerytrhritol, des alcools de sucre, des alkylglucosides, ainsi que des polyglucosides ; des mono-, di- , et trialkylphosphates ainsi que des alkylpphosphates de mono-, di- et/ou tri PEG et leurs sels ; des alcools de cire de laine ; des copolymères de polysiloxane-polyalkyle-polyéther ; des éthers mixtes à base de pentaerythritol, acides gras, acide citrique et alcool gras et/ou des esters mixtes d'acides gras ayant de 6 à 22 atomes de carbone, de méthylglucose et de polyols ainsi que des polyalkylèneglycols.

3. Compositions selon l'une quelconque des revendications 1 à 2,
**caractérisées en ce qu'**
elles contiennent des agents émulsionnants ioniques qui sont choisis dans le groupe formé des alkylethersulfates, des sulfates de monoglycérides d'acide gras, des sulfates d'esters d'acide gras, des isethionates d'acide gras, des produits de condensation de protéines et d'acides gras, des bétaïnes et des esterquats.

4. Utilisation d'ester d'acide carboxylique plurifonctionnel et/ou fonctionnalisé par un hydroxy choisi dans le groupe de l'acide succinique, de l'acide maléique, de l'acide itaconique, de l'acide adipique, de l'acide sébacique, de l'acide dodécanedioïque, et/ou de l'acide malique, de l'acide tartrique, et d'alcools gras ayant de 12 à 18 atomes de carbone en tant que composés huileux dans des compositions de nettoyage de la peau.
